# EUROPEAN PATENT SPECIFICATION

(11) **EP 1 586 901 B1**
(45) Date of publication and mention of the grant of the patent: **13.06.2012**
(21) Application number: 04792597.9
(22) Date of filing: 19.10.2004
(51) Int. Cl.: G01N 33/52, G01N 33/543, G06K 19/06

(54) **TOOL FOR ANALYZING SPECIMEN**
WERKZEUG ZUM TESTEN EINER PROBE
OUTIL D'ANALYSE D'ECHANTILLON

(30) Priority: 20.10.2003 JP 2003359713; 20.10.2003 JP 2003359714
(43) Date of publication of application: 19.10.2005
(73) Proprietor: ARKRAY, Inc., Kyoto 601-8045 (JP)
(72) Inventor: SAKAMOTO, Hisashi c/o Arkray, Inc., Kyoto-shi Kyoto 601-8045 (JP); MATSUDA, Takeshi c/o Arkray, Inc., Kyoto-shi Kyoto 601-8045 (JP); EGAWA, Kouji c/o Arkray, Inc., Kyoto-shi Kyoto 601-8045 (JP); SAIJI, Tetsuaki c/o Arkray, Inc., Kyoto-shi Kyoto 601-8045 (JP)
(74) Representative: Hall, Matthew Benjamin
(86) International application number: PCT/JP2004/015428
(87) International publication number: WO 2005/038456

(56) References cited:
- EP-A- 0 590 587
- EP-A- 0 837 320
- WO-A-01/13329
- WO-A-02/088739
- JP-A- 2001 349 835
- JP-A- 2002 228 658
- JP-A- 2003 247 992
- US-A- 3 907 503
- US-A1- 2002 028 015

## Description

### Technical Field

The present invention relates to a sample analysis tool.

### Background Art

In clinical tests, a sample analysis tool in which a pad(s) impregnated with a reagent(s) is disposed on a plastic substrate has been used for various purposes. For example, in a sample analysis tool used for a urinalysis, pads respectively impregnated with reagents for test items such as glucose (GLU), protein (PRO), bilirubin (BIL), urobilinogen (URO), pH, occult blood (BLD), ketone bodies (KET), nitrite (NIT), leukocytes (LEU), S.G. (specific gravity), and a color tone are disposed on a plastic substrate. These items can be tested by impregnating the respective pads of this sample analysis tool with urine and then measuring a change in color tone of each reagent either visually or with an optical analyzer. When measuring the change in color tone with an optical analyzer, a predetermined tube containing a urine sample is set in the analyzer together with the sample analysis tool, thus allowing the analysis to be conducted automatically or semiautomatically. Note here that such a sample analysis tool also may be called a test piece or a test paper.

A sample analysis tool to be analyzed by an analyzer may include a black marker, a plurality of color markers, a bar code or the like to provide information as to the type of the sample analysis tool, the production batch from which the sample analysis tool is derived, the provider of the sample analysis tool, etc. to an analyzer (see Patent Document 1, Patent Document 2, Patent Document 3, and Patent Document 4, for example). However, a sample analysis tool including a black marker can provide only a small amount of information because it only provides two pieces of information by the presence or absence of the black marker. Furthermore, a method using a plurality of color markers has a problem in that unless the color markers used in the sample analysis tool are suitable for the measurement wavelength of a light source of the analyzer, information may not be detected due to the difference in type of the analyzer or in production batch of the color markers. Moreover, although a sample analysis tool including a bar code can provide a sufficient amount of information, it requires the analyzer to be provided with a bar code reader, which leads to an increase in cost and complexity of the analyzer, for example.
Patent Document 1: JP 51(1976)-23791 A
Patent Document 2: JP 50(1975)-104991 AAlso published as US 3907503 (D2)
Patent Document 3: JP 10(1998)-132734 A - Also published as EP 0837320 (D1)
Patent Document 4: U.S. Patent. No. 4,592,893

### Disclosure of Invention

### Problem to be Solved by the Invention

It is an object of the present invention to provide a sample analysis tool that can provide a sufficient amount of information accurately and allows an analyzer to read out the information without using any special mechanism.

### Means for Solving Problem

In order to achieve the above object, the present invention provides a sample analysis tool including a substrate, reagent layers, and an information recording layer. The regent layers, and the information recording layer are formed on the substrate. The information recording layer is composed of one region or divided into a plurality of regions, and each region is colored with a single color. Information is recorded using the position of reagent layers and one selected from the group consisting of the single color of each region, a combination of color intensities over the plurality of regions, and an area of each region, and the recorded information can be read out optically. There are a plurality of reagent layers arranged in series, pitches between the plurality of reagent layers are constant, except for at least one of a first pitch, a last pitch, and a pitch present between the first pitch and the last pitch, and this variation in pitch is also used to record the information.

### Effects of the Invention

As descried above, in the sample analysis tool of the present invention, the information recording layer serves to provide a sufficient amount of information to an analyzer accurately. Besides, since the information can be read out with an ordinary optical system for measurement, it is not necessary to provide the analyzer with any special mechanism.

### Brief Description of Drawings

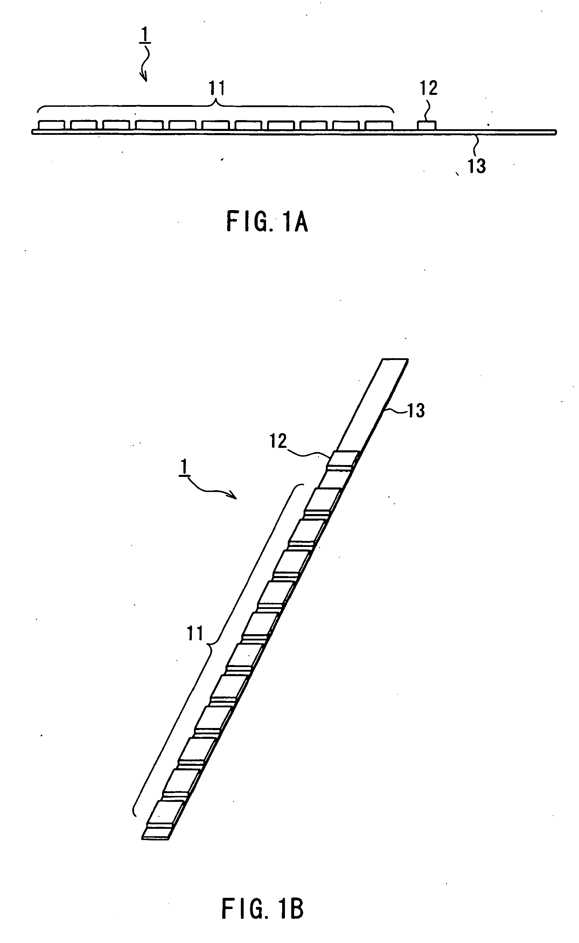

[0007] [FIG. 1] FIGs. 1A and 1B are a side view and a perspective view, respectively, showing an example of a sample analysis tool.
[FIG. 2] FIG. 2 shows an example of information recording in a sample analysis tool.
[FIG. 3] FIG. 3 shows another example of information recording in a sample analysis tool.
[FIG. 4] FIG. 4 shows still another example of information recording in a sample analysis tool.
[FIG. 5] FIGs. 5A and 5B are a side view and a perspective view, respectively, showing an example of a sample analysis tool of the present invention.
[FIG. 6] FIG. 6 shows another example of information recording in a sample analysis tool of the present invention.
[FIG. 7] FIG. 7 shows still another example of information recording in a sample analysis tool of the present invention.
[FIG. 8] FIG. 8 shows an example of an information recording layer of a sample analysis tool of the present invention.
[FIG. 9] FIG. 9 shows another example of an information recording layer of a sample analysis tool of the present invention.
[FIG. 10] FIG. 10 shows still another example of an information recording layer of a sample analysis tool of the present invention.
[FIG. 11] FIG. 11 shows still another example of an information recording layer of a sample analysis tool of the present invention.
[FIG. 12] FIG. 12 shows still another example of an information recording layer of a sample analysis tool of the present invention.
[FIG. 13] FIG. 13 shows an example where an information recording part is formed on a base film by printing.
[FIG. 14] FIG. 14 shows still another example of a sample analysis tool of the present invention.

### Explanation of reference numerals

1: sample analysis tool
11: reagent layer
12: information recording layer
13: substrate
121: information recording part
131: base film

### Description of the Invention

In the following, the present invention will be described in further detail.

The sample analysis tool of the present invention is configured so that the information is recorded using at least one of the single color of each region, the combination of color intensities over the plurality of regions, and the area of each region and a position of the reagent layer, and the recorded information can be read out optically. A plurality of reagent layers are provided. Pitches between the plurality of reagent layers are constant, except for at least one of a first pitch, a last pitch, and a pitch present between the first pitch and the last pitch, and this variation in pitch also is used to record the information.

In the sample analysis tool of the present invention, the single color is not particularly limited, and may be, for example, a chromatic color such as yellow (an absorption wavelength: 350 nm or more and less than 430 nm), blackish brown, red (an absorption wavelength: 430 nm or more and less than 530 nm), purple (an absorption wavelength: 530 nm or more and less than 580 nm), blue (an absorption wavelength: 580 nm or more and less than 640 nm), or green (an absorption wavelength: 640 nm or more and 800 nm or less). Apart from the chromatic color, the single color may be an achromatic color such as white, gray, or black. The intensity of the single color may be set so as to change over a plurality of steps or change continuously so as to exhibit color gradation as will be described later. In the case where the intensity of the single color is set to a plurality of levels, the number of levels may be, for example, 1 to 10, preferably 1 to 6, more preferably 1 to 4, and still more preferably 3 or 4. Examples of the intensity levels of the single color include intensity levels of achromatic colors such as white, gray, and black. On the other hand, in the case of chromatic colors, the intensity of yellow, blackish brown, red, purple, blue, green, or the like can be set to a plurality of levels by setting their light absorption or light reflection to a plurality of levels, for example. More specifically, the light absorption or the light reflection of each color can be set to so as to change stepwise with the wavelength specific thereto remaining substantially unchanged. In the case where the light absorption or the light reflection is set to a plurality of levels, the color may be regarded as having a high intensity when it exhibits an absorbance or a reflectance of 100% to 80%, as having an intermediate intensity when it exhibits an absorbance or a reflectance of 70% to 40%, and as having a low intensity when it exhibits a an absorbance or a reflectance of 20% to 5%, for example. It is to be noted that, in the present invention, the term "single color" refers to, in the case of a chromatic color, a color that exhibits light absorption or light reflection (including 100% and 0% absorption or reflection) within a wavelength range specific thereto, and the intensity of the color refers to the degree of the light absorption or light reflection. On the other hand, in the case of an achromatic color, the term "single color" refers to a color whose absorbance or reflectance varies in the state where light absorption or light reflection (including 100% and 0% absorption or reflection) remains substantially the same over the entire wavelength range from 300 nm to 900 nm. In the present invention, the intensity of a single color can be measured with an optical system generally provided in an analyzer, but another optical system may be provided in the analyzer separately. For example, the optical system may be attached to a nozzle that is used for dropping a sample droplet because the nozzle moves above the surface of the sample analysis tool. Alternatively, the optical system may be attached to a drum of a feeder that is used for taking out the sample analysis tool automatically. Examples of means for carrying out optical measurement include measuring a reflectance and measuring an absorbance.

In the sample analysis tool of the present invention, the number of regions included in the information recording layer is not particularly limited, and may be, for example, 8 to 10, preferably 4 to 6, and more preferably 2 or 3.

In the sample analysis tool of the present invention, it is preferable that there are two or more information recording layers. This allows a still greater amount of information to be recorded. The number of information recording layers is not particularly limited, and may be 1 to 10, preferably 1 to 5, more preferably 1 to 3. When the sample analysis tool of the present invention includes a plurality of information recording layers, it is preferable that at least one of the two or more information recording layers is formed between the reagent layers. With this configuration, it is possible to increase an amount of information to be recorded in the sample analysis tool while utilizing a space on a substrate surface effectively.

With regard to the combination of color intensities over the plurality of regions, a change in intensity of the single color between adjacent ones of the plurality of regions is not particularly limited, and may be a continuous change, a stepwise change, or a combination of a continuous change and a stepwise change. More specifically, a change in intensity of the single color over the plurality of regions may be a stepwise change in which an intensity of a single color in one region is distinctly different from that in the region adjacent thereto or may be a continuous change in which an intensity of a single color in one region shifts gradually to that in the region adjacent thereto. Also, both the changes may be used in combination. In the case of a continuous change, the optical measurement preferably is the measurement of a change ratio (or a duty ratio) of an intensity of reflected light or an absorbance, rather than the measurement of an absolute reflectance or absorbance. For example, in the case where information is recorded using an intensity of an achromatic color that changes stepwise so as to exhibit white, black, and gray, the dynamic range is too broad so that, in the case of a simple optical sensor, optical calibration is required to recognize gray. However, when the intensity of the achromatic color changes continuously (so as to exhibit color gradation), it is possible to read out information easily even in the presence of gray by measuring a change in reflectance or absorbance. Moreover, even in the case where the intensity of a single color changes stepwise so as to be distinctly visible, it is also possible to read out information based on a change in absorbance or reflectance.

In the sample analysis tool of the present invention, there may be two or more information recording layers that are in contact with each other, and an intensity of the single color may change continuously between adjacent ones of the information recording layers. In this case also, information can be recorded and read out in the same manner as in the above.

In the sample analysis tool of the present invention, the number of reagent layers is not particularly limited, and may be, for example, 2 to 15, preferably 2 to 10, and more preferably 2 to 8. One example of information recording using the arrangement of the reagent layers is as follows. A plurality of reagent layers are formed so as to be arranged in series. Pitches between the plurality of reagent layers are constant, except for at least one of a first pitch, a last pitch, and a pitch present between the first pitch and the last pitch. This variation in pitch is used to record information. More specifically, given that the reagent layers are arranged in series at a constant pitch on the substrate, it is possible to form a varied pitch by not providing a reagent layer at a position where it is supposed to be. In this case, by setting the state where a reagent layer is provided at a position where it is supposed to be to [1] and the state where a reagent layer is not provided at a position where it is supposed to be to [0], it is possible to form binary digital information using [1] and [0]. In the present invention, the number of reagent layers and the positions of the reagent layers can be determined by, for example, measuring a reflectance or an absorbance with an optical system generally provided in an analyzer.

In the sample analysis tool of the present invention, information recorded in the information recording layer is not particularly limited, and examples thereof include:
- information for automatically identifying a provider of the sample analysis tool;
- information for automatically identifying a country in which the sample analysis tool is sold;
- information for automatically identifying the number of test items and an order in which the test items are arranged;
- information as to a production batch of the sample analysis tool (a calibration curve or information as to a sensibility of the sample analysis tool);
- information as to whether the sample analysis tool is applicable to visual observation only, reading by an analyzer only, or both the visual observation and the reading by an analyzer;
- information for identifying a front side (i.e., a reagent side) and a rear side of the sample analysis tool;
- information for identifying the presence or absence of a reagent in the sample analysis tool;
- information for detecting and correcting a positional deviation of the sample analysis tool in an analyzer; and
- information for correcting an amount of light when the sample analysis tool is subjected to measurement with an optical system.

There is no particular limitation on the use of the sample analysis tool of the present invention. For example, the sample analysis tool of the present invention may be used for a urinalysis, a biochemical test, a microorganism test, an immunological test, a genetic analysis, an environmental test, a test for an agricultural chemical, an allergen test, or the like. In the case of a urinalysis, examples of a test item include glucose (GLU), protein (PRO), bilirubin (BIL), urobilinogen (URO), pH, occult blood (BLD), ketone bodies (KET), nitrite (NIT), leukocytes (LEU), S.G. (specific gravity), a color tone, ascorbic acid, a salt concentration, highly-sensitive protein, albumin, creatinine, Bence-Jones protein, hormones and physiologically active substances. In the sample analysis tool capable of dealing with a plurality of test items, at least one reagent layer is provided for each of the test items.

The sample analysis tool of the present invention preferably is analyzed by an analyzer, but may be subjected to visual observation. Moreover, there is no particularly limitation on the type of the analyzer, and the analyzer may be operated manually, semiautomatically, or automatically.

Hereinafter, examples of the sample analysis tool according to the present invention will be described with reference to the accompanying drawings. Note here that in FIGs. 1 to 14, the same components are denoted with the same reference numerals.

### Example 1

FIG. 1 shows an example of a sample analysis tool. FIG. 1A is a side view of the sample analysis tool and FIG. 1B is a perspective view of the same. As shown in FIG. 1, in this sample analysis tool 1, eleven reagent layers 11 and one information recording layer 12 are formed on a strip-shaped substrate 13. The material of the substrate is not particularly limited, and may be, for example, a resin, metal, glass or the like. Also, the color of the substrate is not particularly limited, and may be white, gray, black, a chromatic color, or transparent. Furthermore, the size of the substrate is not particularly limited, and may be determined as appropriate depending on a test item, the standard of an analyzer to be used, etc. For example, the substrate may be 50 to 150 mm in length, 2 to 10 mm in width, and 0.1 to 1.0 mm in thickness. Each of the reagent layers 11 is formed by adhering a pad impregnated with a predetermined reagent corresponding to a test item to the substrate 13. The pad may be formed of a filter paper, a glass fiber filter paper, a knitted fabric, a woven fabric, a nonwoven fabric, a membrane filter, a porous resin sheet, or the like, for example. The shape of each reagent layer 11 (pad) is not particularly limited, and may be square, rectangular, circular, oval, or the like. The size of each reagent layer 11 (pad) is not particularly limited. For example, in the case of a square reagent layer (pad), it may be 2 to 10 mm in vertical and horizontal lengths and 0.05 to 1.0 mm in thickness. The number of reagent layers 11 can be increased or decreased in accordance with test items. In the present example, which is not an embodiment of the present invention, eleven reagent layers 11 are arranged in series at a constant pitch. The pitch is not particularly limited, but may be, for example, 1.0 to 100 mm. The information recording layer 12 can be formed by printing or adhering a film, for example. The shape of the information recording layer 12 is not particularly limited, and may be square, rectangular, circular, oval, or the like. The size of the information recording layer 12 is not particularly limited, but when the information recording layer 12 is formed by adhering a square plastic film, the information recording layer 12 may be, for example, 1.0 to 10 mm in vertical and horizontal lengths and 0.05 to 1.0 mm in thickness. The number of information recording layers 12 is not particularly limited, and only one information recording layer 12 may be provided as in the present example or two or more information recording layers 12 may be provided. The distance between the information recording layer 12 and the reagent layer 11 adjacent thereto is not particularly limited, and may be, for example, in the range from 0 (in the case where these layers are in contact with each other) to 100 mm. In this sample analysis tool, a space is provided in one end portion (on the right in FIG. 1A) of the substrate 13 by not providing the reagent layers 11 or the information recording layer 12, so that the sample analysis tool can be handled by pinching this portion with fingertips.

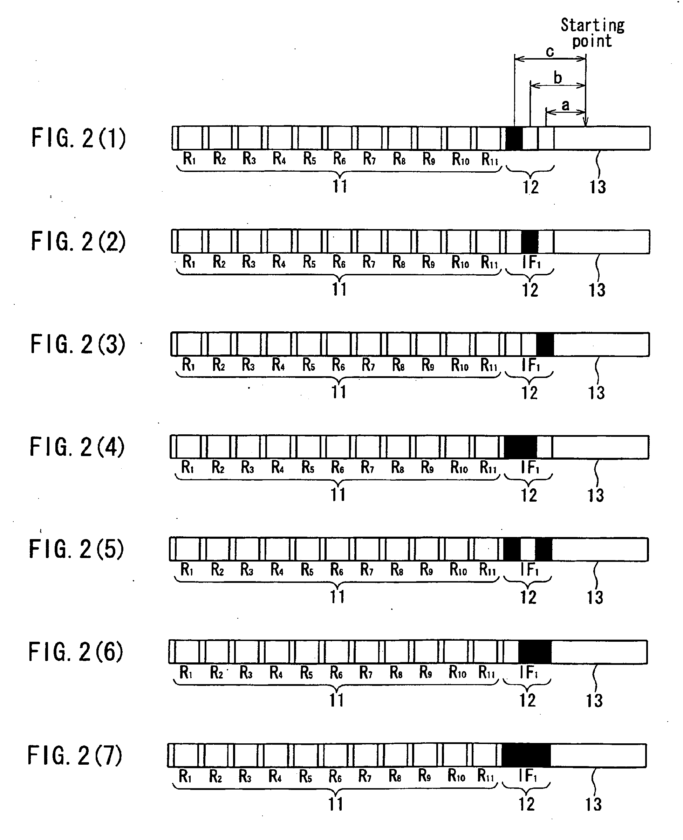

Next, examples of information recording in this sample analysis tool will be described with reference to FIG. 2, FIG. 3 and FIG. 4. In these drawings, the same components as those in FIG. 1 are denoted with the same reference numerals as those therein. In the following, as examples of an intensity of a single color, intensities of white, gray, black, and red are shown. However, the present invention is not limited thereto, and intensities of chromatic colors such as yellow (an absorption wavelength: 350 nm or more and less than 430 nm), blackish brown, purple (an absorption wavelength: 530 nm or more and less than 580 nm), blue (an absorption wavelength: 580 nm or more and less than 640 nm), and green (an absorption wavelength: 640 nm or more and 800 nm or less) also may be used.

FIG. 2 is a plan view showing an example of the sample analysis tool. In FIG. 2, numerals R1 to R11 denote the reagent layers, respectively, and numeral IF1 denotes the information recording layer 12. As shown in FIG. 2, the information recording layer 12 of this sample analysis tool 1 is divided into three rectangular regions, and white and black are used to record information. In the case where the information recording layer 12 includes three regions and two achromatic colors, namely, white and black, are used, the number of possible combinations is 2 × 2 × 2 so that eight types of information can be recorded. In FIG. 2, (1) to (7) respectively show examples where seven types of information are recorded. The recorded information can be read out with an optical system provided in an analyzer without using any special device.

### (Information Example 1)

In the following, an example of a process by which an analyzer identifies and retrieves various types of information recorded in the sample analysis tool will be described specifically.
(1) A sample is supplied to each of the reagent layers 11 of the sample analysis tool by, for example, dropping a sample droplet or by dipping.
(2) The sample analysis tool 1 is placed at a predetermined position of a designated sample analysis tool holder of the analyzer with the reagent layers 11 of the sample analysis tool 1 facing up.
(3) The optical system of the analyzer moves to a predetermined position (a starting position).
(4) The optical system starts to scan the surface of the sample analysis tool sequentially to measure a distance from the starting position and a reflectance.
(5) The optical system measures reflectances at all the wavelengths (e.g., 400, 450, 500, 550, 630, and 750 nm) every time it moves a certain distance.
(6) Based on the positions of the respective divided regions of the information recording layer 12 (IF1) and the amounts of signals at all the wavelengths, the analyzer retrieves the information recorded in the sample analysis tool 1 automatically.
(7) Based on the information thus retrieved, the analyzer performs appropriated operations, actions, and analyses.

### (Information Example 2)

Next, taking the sample analysis tool shown in (1) of FIG. 2 as an example, an example of how the information recorded in IF1 is retrieved and how the sample is analyzed base on this information will be described in the following.
(1) The optical system of the analyzer starts to scan the surface of the sample analysis tool from the starting position. At a portion apart from the starting position by the distance "a", the optical system identifies the portion as white when reflectances at all the wavelengths fall within the range from 90% to 100%.
(2) At a portion apart from the starting position by the distance "b", the optical system identifies the portion as white when reflectances at all the wavelengths fall within the range from 90% to 100%.
(3) At a portion apart from the starting position by the distance "c", the optical system identifies the portion as black when reflectances at all the wavelengths fall within the range from 5% to 20%.
(4) Information recorded in the sample analysis tool is read out by the identifications described in (1) to (3) above, and the information thus read out is then compared with information stored in the analyzer. For example, the number of test items to be tested by the reagent layers (ten items in (1) of FIG. 2) and the positions of the respective reagent layers are compared with the information stored in the analyzer so as to determine whether or not they agree with each other. In the case where they do not agree with each other, it is recognized as an error. During the above-described identifications, it is also possible to determine whether or not the sample analysis tool is set in a predetermined position in the analyzer and whether or not the sample analysis tool is set in an appropriate orientation (i.e., whether or not the sample analysis tool is arranged with the reagent layer side facing up).
(5) The optical system identifies a midpoint of the black marker located at a position apart from the starting position by the distance "c" or a position of an edge of the black marker, and then reads out information as to a position of a midpoint of each reagent layer relative to this position. Based on the thus-obtained position information, the optical system can measure reflectances at the midpoint of each reagent layer. That is, even in the case where the sample analysis tool is not placed at a predetermined position of the predetermined part (the holder) of the analyzer, it is possible to calculate the position of each reagent layer relative to the black marker. Therefore, even if the sample analysis tool is displaced in the holder, the position of each reagent layer can be recognized and measured accurately.

### (Information Example 3)

Information Example 1 described above is directed to the case where a portion apart from the starting position by the distance "c" is colored black. In the following, an example where different types of information are recorded by coloring the above portion either gray with various intensities or white.
(1) At a portion apart from the starting position by the distance "c", the optical system identifies the portion as dark gray when reflectances at all the wavelengths fall within the range from 20% to 30%. This provides information indicating that there are reagent layers corresponding to nine test items, the positions of the reagent layers, and that the sample analysis tool is placed with the reagent layer side facing up.
(2) At a portion apart from the starting position by the distance "c", the optical system identifies the portion as gray with an intermediate intensity when reflectances at all the wavelengths fall within the range from 30% to 50%. This provides information indicating that there are reagent layers corresponding to eight test items, the positions of the reagent layers, and that the sample analysis tool is placed with the reagent layer side facing up.
(3) At a portion apart from the starting position by the distance "c", the optical system identifies the portion as light gray when reflectances at all the wavelengths fall within the range from 50% to 80%. This provides information indicating that there are reagent layers corresponding to seven test items, the positions of the reagent layers, and that the sample analysis tool is placed with the reagent layer side facing up.
(4) At a portion apart from the starting position by the distance "c", the optical system identifies the portion as white when reflectances at all the wavelengths fall within the range from 90% to 100%. This provides information indicating that the sample analysis tool is placed with the reagent layer side facing down. In this case, the analyzer recognizes that the sample analysis tool is not set in the analyzer appropriately and displays an error message.

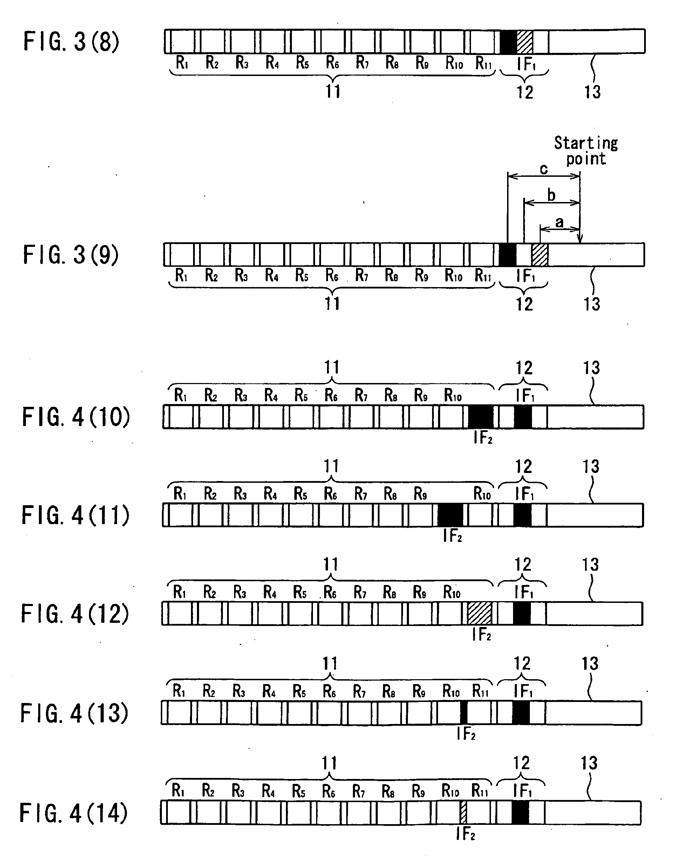

In Information Examples 2 and 3 described above, five types of information are recorded at a portion apart from the starting position by the distance "c" using black, gray with various intensities, and white. Other than this, it is also possible to record information using a combination of color intensities at a portion apart from the starting position by the distance "a" and a portion apart from the starting position by the distance "b" (see (2) to (7) in FIG. 2 and (8) and (9) in FIG. 3). Also, not only a combination of intensities of black but also combinations of intensities of yellow, blackish brown, red, purple, blue, and green can be used to record information as to the sample analysis tool. Although IF1 is divided into three regions in the above examples, IF1 may be divided into four or more regions.

### (Information Example 4)

FIG. 3 shows another example of information recording in the sample analysis tool. In FIG. 3, numerals R1 to R11 denote the reagent layers, respectively, and numeral IF1 denotes the information recording layer 12. As shown in FIG. 3, the information recording layer 12 of this sample analysis tool 1 is divided into three rectangular regions, and white, black, and gray are used to record information. In the case where the information recording layer 12 include three regions and three types of color intensities are used, the number of possible combinations is 3 × 3 × 3 so that 27 types of information can be recorded. In FIG. 3, (8) and (9) respectively show examples where two types of information are recorded in the information recording layer. The recorded information can be read out with an optical system provided in an analyzer without using any special device.

### (Information Example 5)

Taking the sample analysis tool shown in (9) of FIG. 3 as an example, an example of how the information is recorded using a chromatic color and how the recorded information is read out will be described.
(1) In this sample analysis tool ((9) of FIG. 3), a portion apart from the starting position by the distance "a" is colored pink, a portion apart from the starting position by the distance "b" is colored white, and a portion apart from the starting position by the distance "c" is colored red.
(2) The optical system starts the scanning, and at the portion apart from the starting position by the distance "a", the optical system identifies the portion as pink when a reflectance at a wavelength of 500 nm falls within the range from 40% to 60% and reflectances at other wavelengths (400, 450, 550, 630, and 750 nm) are all greater than the reflectance at 500 nm falling within the range from 40% to 60% so that the reflectance at 500 nm is the smallest.
(3) At a portion apart from the starting position by the distance "b", the optical system identifies the portion as white when reflectances at all the wavelengths fall within the range from 80% to 100%.
(4) At the portion apart from the starting position by the distance "c", the optical system identifies the portion as red when a reflectance at a wavelength of 500 nm falls within the range from 5% to 20% and reflectances at other wavelengths (400, 450, 550, 630, and 750 nm) are all greater than the reflectance at 500 nm falling within the range from 5% to 20% so that the reflectance at 500 nm is the smallest.
(5) According to the above-described steps, it is possible to read out the information recorded in the information recording layer 12. For example, it is possible to read out information as to the type of the reagents, the number of test items, the orientation of the sample analysis tool (whether the reagents layer side faces up or down), etc., as in the cases of Information Examples 1 to 5 described above,
(6) The optical system identifies a midpoint of a red marker located at a portion apart from the starting position by the distance "c" or a position of an edge of the red marker. The analyzer stores a position of a midpoint of each reagent layer relative to this position. Thus, the optical system can measure reflectances at the midpoint of each reagent layer. Accordingly, even in the case where the sample analysis tool is not placed at a predetermined position of the holder of the analyzer, it is possible to calculate the distance from the red marker to each reagent layer. Therefore, even if the sample analysis tool is displaced in the holder, the position of each reagent layer can be recognized and measured accurately.

FIG. 4 is a plan view showing still another example of the sample analysis tool. In FIG. 4, numerals R1 to R11 denote the reagent layers, respectively, and numerals IF1 and IF2 denote information recording layers 12. As shown in FIG. 4, IF1 as one of the information recording layers 12 of this sample analysis tool 1 is divided into three rectangular regions, and white and black are used to record information. Furthermore, in (10), (11), and (12) of FIG. 4, there are only ten reagent layers 11 with one reagent layer provided in the above-described examples is missing, and in place of this missing reagent layer, IF2 is formed as another information recording layer 12. On the other hand, in (13) and (14) of FIG. 4, IF2 is formed as another information recording layer 12 in a region between R10 and R11 as the reagent layers 11.

### Example 2

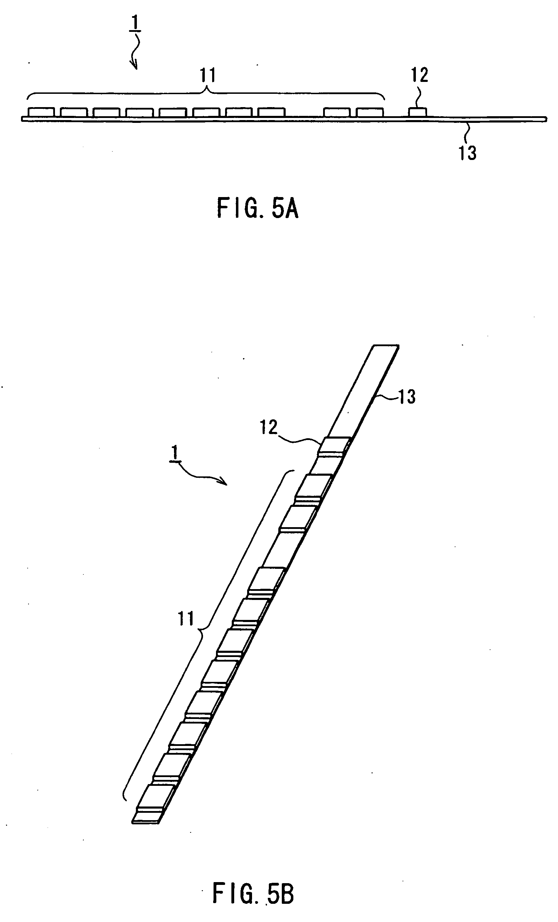

FIG. 5 shows an example of the sample analysis tool according to the present invention. FIG. 5A is a side view of the sample analysis tool and FIG. 5B is a perspective view of the same. As shown in FIG. 5, in this sample analysis tool 1, ten reagent layers 11 and one information recording layer 12 are formed on a strip-shaped substrate 13. The material of the substrate is not particularly limited, and may be, for example, a resin, metal, glass or the like. Also, the color of the substrate is not particularly limited, and may be white, gray, black, a chromatic color, or transparent. Furthermore, the size of the substrate is not particularly limited, and may be determined as appropriate depending on a test item, the standard of an analyzer to be used, etc. For example, the substrate may be 50 to 150 mm in length, 2 to 10 mm in width, and 0.1 to 1.0 mm in thickness. Each of the reagent layers 11 is formed by adhering a pad impregnated with a predetermined reagent corresponding to a test item to the substrate 13. The pad may be formed of a filter paper, a glass fiber filter paper, a knitted fabric, a woven fabric, a nonwoven fabric, a membrane filter, a porous resin sheet, or the like, for example. The shape of each reagent layer 11 (pad) is not particularly limited, and may be square, rectangular, circular, oval, or the like. The size of each reagent layer 11 (pad) is not particularly limited. For example, in the case of a square reagent layer (pad), it may be 2 to 10 mm in vertical and horizontal lengths and 0.05 to 1.0 mm in thickness. The number of reagent layers 11 can be increased or decreased in accordance with test items. These reagent layers 11 are arranged at a constant pitch that is determined based on the assumption that eleven reagent layers should be arranged in series, but one reagent layer is missing in the middle of the row of the reagent layers 11 (between the eighth and ninth reagent layers from the left in FIG. 5A). This is used to record information. The pitch is not particularly limited, but may be, for example, 1.0 to 100 mm. The information recording layer 12 can be formed by printing or adhering a film, for example. The shape of the information recording layer 12 is not particularly limited, and may be square, rectangular, circular, oval, or the like. The size of the information recording layer 12 is not particularly limited, but when the information recording layer 12 is formed by adhering a square plastic film, the information recording layer 12 may be, for example, 1.0 to 10 mm in vertical and horizontal lengths and 0.05 to 1.0 mm in thickness. The number of information recording layers 12 is not particularly limited, and only one information recording layer 12 may be provided as in the present example or two or more information recording layers 12 may be provided. The distance between the information recording layer 12 and the reagent layer 11 adjacent thereto is not particularly limited, and may be, for example, in the range from 0 (in the case where these layers are in contact with each other) to 100 mm. In this sample analysis tool, a space is provided in one end portion (on the right in FIG. 5A) of the substrate 13 by not providing the reagent layers 11 or the information recording layer 12, so that the sample analysis tool can be handled by pinching this portion with fingertips.

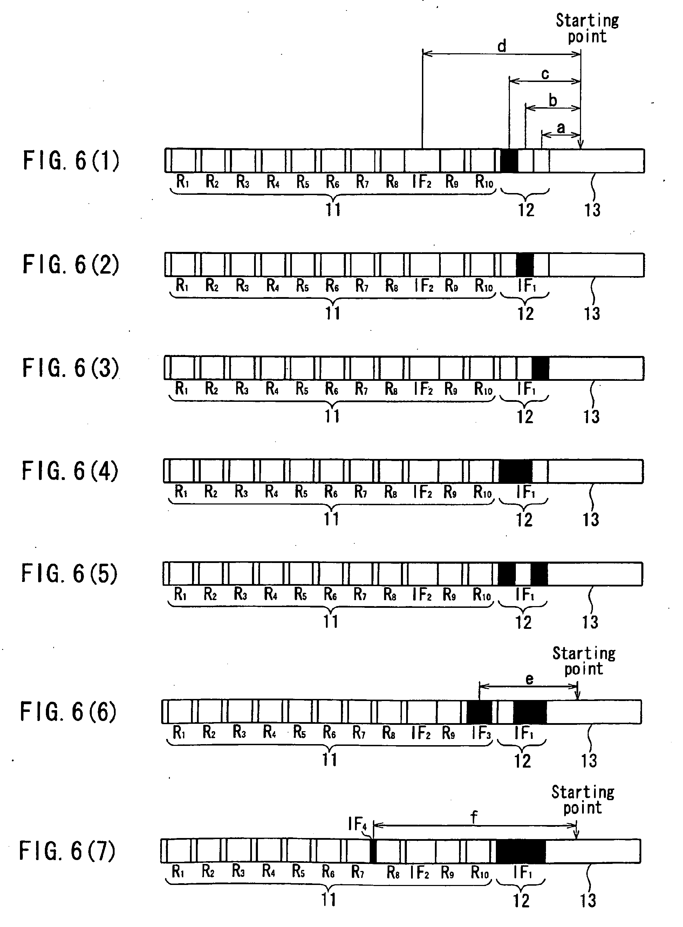

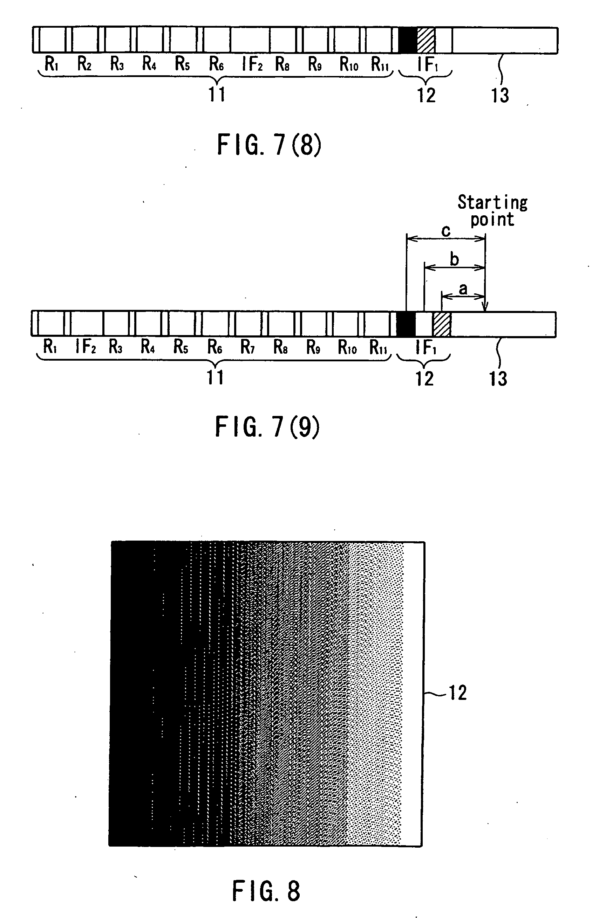

Next, examples of information recording in this sample analysis tool will be described with reference to FIG. 6 and FIG. 7. In these drawings, the same components as those in FIG. 5 are denoted with the same reference numerals as those therein. In the following, as examples of an intensity of a single color, intensities of white, gray, black, and red are shown. However, the present invention is not limited thereto, and intensities of chromatic colors such as yellow (an absorption wavelength: 350 nm or more and less than 430 nm), blackish brown, purple (an absorption wavelength: 530 nm or more and less than 580 nm), blue (an absorption wavelength: 580 nm or more and less than 640 nm), and green (an absorption wavelength: 640 nm or more and 800 nm or less) also may be used.

As shown in FIG. 6, the information recording layer 12 of this sample analysis tool is divided into three regions, and white and black are used to record information. In the case where the information recording layer 12 includes three regions and two colors, namely, white and black, are used, the number of possible combinations is 2 × 2 × 2 so that eight types of information can be recorded. In FIG. 6, (1) to (7) respectively show examples where seven types of information are recorded in the information recording layer 12.

In FIG. 6, numerals R1 to R10 denote the reagent layers corresponding to ten test items at most. IF1 denotes the information recording layer 12, and IF2 denotes a portion where information is recorded by removing one reagent layer out of the reagent layers arranged in series, thus forming an "empty pitch". IF3 denotes a portion where information is recorded by coloring another "empty pitch" formed by removing a reagent layer with a single color. The coloring can be achieved by printing or by adhering a color film or a color tape. IF4 denotes a portion where information is recorded by coloring a surface of the substrate between the reagent layers with a single color. The coloring can be achieved by printing or by adhering a color film or a color tape. Although not shown in the drawing, it is also possible to record information by coloring a rear surface of the substrate with a single color in the same manner as in the above (this portion is referred to as IF5). The coloring can be achieved by printing or by adhering a color film or a color tape. By using these information recording portions IF1 to IF5 in combination, the sample analysis tool can provide a large amount of information.

Although a single empty pitch is used to provide IF2 in the sample analysis tool shown in FIG. 6, a plurality of empty pitches may be used to provide IF2 in order to increase an amount of information recordable therein. Furthermore, since each of the reagent layers denoted with R1 to R11 has a thickness, each of the reagent layers alone can contain information as to the sample analysis tool. For example, since the height of each of the reagent layers is different from that of the substrate, a reflected signal and a transmitted signal from each of the reagent layers are different from those from the base owing to the difference in height. This can be utilized for information recording. Moreover, a still more abrupt change in signal occurs at edge portions of each of the reagent layers. Thus, such signals can be used as information as to the number of reagent layers.

According to the method in which a large amount of information is recorded using a combination of intensities of a single color in a simple manner and the method in which information is retrieved based on a structural feature of the sample analysis tool (e.g., the arrangement of the reagent layers) as described above, an analyzer can read out the recorded information with an optical system provided therein for measuring color development in the reagent layers without using any special device. Moreover, by further providing a simple optical system in the analyzer, it becomes possible to retrieve a large amount of information as to the reagents and the analyzer.

### (Information Example 2-1)

In the following, an example of a process by which an analyzer identifies and retrieves various types of information recorded in the sample analysis tool will be describe specifically.
(1) sample is supplied to each of the reagent layers 11 of the sample analysis tool by, for example, dropping a sample droplet or by dipping.
(2) The sample analysis tool 1 is placed at a predetermined position of a designated sample analysis tool holder of the analyzer with the reagent layers 11 of the sample analysis tool 1 facing up.
(3) The optical system of the analyzer moves to a predetermined position (a starting position).
(4) The optical system starts to scan the surface of the sample analysis tool sequentially to measure a distance from the starting position and a reflectance.
(5) The optical system measures reflectances at all the wavelengths (e.g., 400, 450, 500, 550, 630, and 750 nm) every time it moves a certain distance.
(6) Based on the positions of the respective divided regions of the information recording layer 12 (IF1) and the amounts of signals at all the wavelengths, the analyzer retrieves the information recorded in the sample analysis tool 1 automatically.
(7) The positions of the reagent layers (R1 to R10 etc.) and IF2, IF3, IF4, and IF5 (the information recording portion on the rear side of the substrate) and reflectances at portions with various single color intensities are measured, and the analyzer automatically retrieves the information recorded in the sample analysis tool 1.
(8) Based on the information thus retrieved, the analyzer performs appropriated operations, actions, and analyses.

### (Information Example 2-2)

Next, taking the sample analysis tool shown in (1) of FIG. 6 as an example, an example of how the information recorded in IF1 is retrieved and how the sample is analyzed based on this information will be described in the following.
(1) The optical system of the analyzer starts to scan the surface of the sample analysis tool from the starting position. At a portion apart from the starting position by the distance "a", the optical system identifies the portion as white when reflectances at all the wavelengths fall within the range from 90% to 100%.
(2) At a portion apart from the starting position by the distance "b", the optical system identifies the portion as white when reflectances at all the wavelengths fall within the range from 90% to 100%.
(3) At a portion apart from the starting position by the distance "c", the optical system identifies the portion as black when reflectances at all the wavelengths fall within the range from 5% to 20%.
(4) Information recorded in the sample analysis tool is read out by the identifications described in (1) to (3) above, and the information thus read out is then compared with information stored in the analyzer. For example, the number of test items to be tested by the reagent layers (ten items in (1) of FIG. 6), the positions of the respective reagent layers, and information that IF2 is present between the reagent layer R8 and the reagent layer R9 are compared with the information stored in the analyzer so as to determine whether or not they agree with each other. In the case where they do not agree with each other, it is recognized as an error. During the above-described identifications, it is also possible to determine whether or not the sample analysis tool is set in a predetermined position in the analyzer and whether or not the sample analysis tool is set in an appropriate orientation (i.e., whether or not the sample analysis tool is arranged with the reagent layer side facing up).
(5) The optical system identifies a midpoint of the black marker located at a position apart from the starting position by the distance "c" or a position of an edge of the black marker, and then reads out information as to a position of a midpoint of each reagent layer relative to this position. Based on the thus-obtained position information, the optical system can measure reflectances at the midpoint of each reagent layer. That is, even in the case where the sample analysis tool is not placed at a predetermined position of the predetermined part (the holder) of the analyzer, it is possible to calculate the position of each reagent layer relative to the black marker. Therefore, even if the sample analysis tool is displaced in the holder, the position of each reagent layer can be recognized and measured accurately.

### (Information Example 2-3)

Information Example 2-1 described above is directed to the case where a portion apart from the starting position by the distance "c" is colored black. In the following, an example where different types of information are recorded by coloring the above portion either gray with various intensities or white.
(1) At a portion apart from the starting position by the distance "c", the optical system identifies the portion as dark gray when reflectances at all the wavelengths fall within the range from 20% to 30%. This provides information indicating that there are reagent layers corresponding to nine test items, the positions of the reagent layers, and that the sample analysis tool is placed with the reagent layer side facing up.
(2) At a portion apart from the starting position by the distance "c", the optical system identifies the portion as gray with an intermediate intensity when reflectances at all the wavelengths fall within the range from 30% to 50%. This provides information indicating that there are reagent layers corresponding to eight test items, the positions of the reagent layers, and that the sample analysis tool is placed with the reagent layer side facing up.
(3) At a portion apart from the starting position by the distance "c", the optical system identifies the portion as light gray when reflectances at all the wavelengths fall within the range from 50% to 80%. This provides information indicating that there are reagent layers corresponding to seven test items, the positions of the reagent layers, and that the sample analysis tool is placed with the reagent layer side facing up.
(4) At a portion apart from the starting position by the distance "c", the optical system identifies the portion as white when reflectances at all the wavelengths fall within the range from 90% to 100%. This provides information indicating that the sample analysis tool is placed with the reagent layer side facing down. In this case, the analyzer recognizes that the sample analysis tool is not set in the analyzer appropriately and displays an error message.

In Information Examples 2-2 and 2-3 described above, five types of information are recorded at a portion apart from the starting position by the distance "c" using black, gray with various intensities, and white. Other than this, it is also possible to record information using a combination of color intensities at a portion apart from the starting position by the distance "a" and a portion apart from the starting position by the distance "b" (see (2) to (7) in FIG. 6 and (8) and (9) in FIG. 7). Also, not only a combination of intensities of black but also combinations of intensities of yellow, blackish brown, red, purple, blue, and green can be used to record information as to the sample analysis tool. Although IF1 is divided into three regions in the above examples, IF1 may be divided into four or more regions.

### (Information Example 2-4)

Next, taking the example of the sample analysis tool shown in (1) of FIG. 6 as an example, an example of how information is recorded using the heights of the reagent layers and the empty pitch (IF2) and how the recorded information is read out will be described.
(1) When the optical system reaches R10 after the start of scanning, the optical system can recognize R10 because reflectances measured at R10 are greater than those measured at the substrate portion owing to the fact that the height of R10 is greater than that of the substrate portion. Based on this, the sample analysis tool can record that the number of reagent layers is ten, and the analyzer can recognize this information.
(2) When both the edge portions of R10 are irradiated with light emitted from the optical system, signals each showing an abrupt change in reflectance are observed. By determined the number of such signals and then dividing the number thus determined by two, the number of reagent layers is obtained. It is possible to record information utilizing this, and the information thus recorded can be read out.
(3) Each of the reagent layers has two edge portions. Based on the distance from the starting position to a midpoint between these two edge portions, it is possible to record and read out information as to a position where each of the reagent layers is adhered to the substrate.
(4) When the optical system reaches a position of the empty pitch (IF2) after the start of scanning and reflectances at all the wavelengths measured at this portion fall within the range from 70% to 100%, the optical system identifies the portion as white. Information that no reagent layer is present at this portion is recorded, and the information thus recorded can be read out.

Although Information Example 2-4 is directed to the case where an empty pitch (IF2) is provided between the reagent layers R8 and R9, the present invention is not limited thereto. IF2 may be provided by removing any one of the reagent layers R1 to R10. Furthermore, by providing a plurality of IF2s, it is possible to record a larger amount of information.

### (Information Example 2-5)

Taking the sample analysis tool shown in (6) of FIG. 6 as an example, an example of a process by which information is recorded in/read out from IF3 will be described. Note here that IF3 denotes a portion of the substrate in which no reagent layer is formed and is colored with a single color.
(1) The optical system starts to scan the surface of the sample analysis tool from the starting position, and at a portion apart from the starting
position by the distance "e", the optical system identifies the portion as black when reflectances at all the wavelengths fall within the range from 5% to 20%.
(2) At a portion apart from the starting position by the distance "e", the optical system identifies the portion as gray with an intermediate intensity when reflectances at all the wavelengths fall within the range from 30% to 50%.
(3) At a portion apart from the starting position by the distance "e", the optical system identifies the portion as white when reflectances at all the wavelengths fall within the range from 90% to 100%, thereby identifying that no reagent layer is present at this portion.

Although Information example 2-5 is directed to an example where the intensity of black is used, it is also possible to record information as to the sample analysis tool using intensities of yellow, blackish brown, red, purple, blue, and green. In this Information Example 2-5, although a portion provided by removing the reagent layer R10 is used as IF3, the present invention is not limited thereto. IF3 may be provided by removing any one of the reagent layers R1 to R10, and only one IF3 or a plurality of IF3s may be provided.

### (Information Example 2-6)

Taking the sample analysis tool shown in (7) of FIG. 6 as an example, an example of a process by which information is recorded in/read out from IF4 will be described. Note here that IF4 denotes a portion of the substrate that is present between the reagent layers and is colored with a single color.
(1) The optical system starts to scan the surface of the sample analysis tool. At a portion (IF4) apart from the starting position by the distance "f", the optical system identifies the portion as black when reflectances at all the wavelengths fall within the range from 5% to 20%.
(2) The optical system starts to scan the surface of the sample analysis tool. At a portion apart from the starting position by the distance "f", the optical system identifies the portion as gray with an intermediate intensity when reflectances at all the wavelengths fall within the range from 30% to 50%.
(3) The optical system starts to scan the surface of the sample analysis tool. At a portion apart from the starting position by the distance "f", the optical system identifies the portion as white when reflectances at all the wavelengths fall within the range from 80% to 100%.

Although Information example 2-6 is directed to an example where the intensity of black is used in IF4, it is also possible to record information as to the sample analysis tool using intensities of yellow, blackish brown, red, purple, blue, and green. In this Information Example 2-6, although a portion between the reagent layers R7 and R8 is used as IF4, the present invention is not limited thereto. IF4 may be provided at any portion between the reagent layers R1 to R10, and only one IF4 or a plurality of IF3s may be provided.

### (Information Example 2-7)

Next, another example of information recording in the sample analysis tool is shown in FIG. 7. In FIG. 7, numerals R1 to R11 denote the reagent layers, IF1 denotes the information recording layer 12, and IF2 denotes a portion where a pitch of the reagent layers arranged in series is varied (i.e., an information recording portion). As shown in FIG. 7, the information recording layer 12 of this sample analysis tool 1 is divided into three rectangular regions, and white, black, and gray are used to record information. In the case where the information recording layer 12 include three regions and three types of color are used, the number of possible combinations is 3 × 3 × 3 so that 27 types of information can be recorded. In FIG. 7, (8) and (9) respectively show examples where two types of information are recorded in the information recording layer. In (8), an arrangement pitch of the reagent layers is varied at a portion denoted with IF2 between the reagent layers R6 and R8. On the other hand, in (9), an arrangement pitch of the reagent layers is varied at a portion denoted with IF2 between the reagent layers R1 and R3. Information is recorded using the variation in the arrangement pitch of the reagent layers. Information also can be recorded using the number of reagent layers. The recorded information can be read out with an optical system provided in an analyzer without using any special device.

### (Information Example 2-8)

Taking the sample analysis tool shown in (9) of FIG. 7 as an example, an example of how information is recorded using a chromatic color and how the recorded information is read out will be described.
(1) In this sample analysis tool ((9) of FIG. 7), a portion apart from the starting position by the distance "a" is colored pink, a portion apart from the starting position by the distance "b" is colored white, and a portion apart from the starting position by the distance "c" is colored red.
(2) The optical system starts the scanning, and at the portion apart from the starting position by the distance "a", the optical system identifies the portion as pink when a reflectance at a wavelength of 500 nm falls within the range from 40% to 60% and reflectances at other wavelengths (400, 450, 550, 630, and 750 nm) are all greater than the reflectance at 500 nm falling within the range from 40% to 60% so that the reflectance at 500 nm is the smallest.
(3) At a portion apart from the starting position by the distance "b", the optical system identifies the portion as white when reflectances at all the wavelengths fall within the range from 80% to 100%.
(4) At the portion apart from the starting position by the distance "c", the optical system identifies the portion as red when a reflectance at a wavelength of 500 nm falls within the range from 5% to 20% and reflectances at other wavelengths (400, 450, 550, 630, and 750 nm) are all greater than the reflectance at 500 nm falling within the range from 5% to 20% so that the reflectance at 500 nm is the smallest.
(5) According to the above-described steps, it is possible to read out the information recorded in the information recording layer 12. For example, it is possible to read out information as to the type of the reagent layers, the number of test items, the orientation of the sample analysis tool (whether the reagents layer side faces up or down), etc., as in the cases of Information Examples 2-1 to 2-7 described above,
(6) The optical system identifies a midpoint of a red marker located at a portion apart from the starting position by the distance "c" or a position of an edge of the red marker. The analyzer stores a position of a midpoint of each reagent layer relative to this position. Thus, the optical system can measure reflectances at the midpoint of each reagent layer. Accordingly, even in the case where the sample analysis tool is not placed at a predetermined position of the holder of the analyzer, it is possible to calculate the distance from the red marker to each reagent layer. Therefore, even if the sample analysis tool is displaced in the holder, the position of each reagent layer can be recognized and measured accurately.

### Example 3

Next, an example where a single color intensity in each of a plurality of regions of an information recording layer is changed continuously will be described with reference to FIGs. 8 to 14. In FIGs. 8 to 14, the same components as those in FIGs. 1 to 7 are denoted with the same reference numerals as those therein.

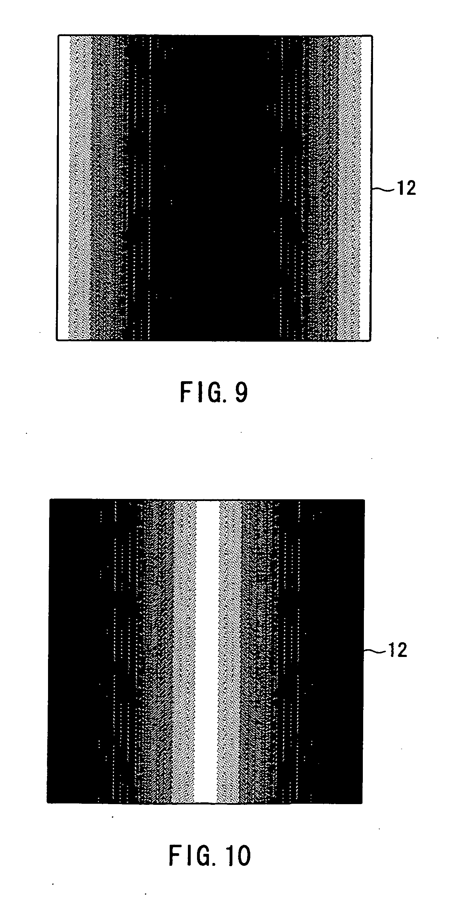

In an information recording layer 12 shown in FIG. 8, from the left toward the right in FIG. 8, an achromatic color changes from black to gray gradually and continuously, and then changes from gray to white gradually and continuously. In an information recording layer 12 shown in FIG. 9, from the left toward the right in FIG. 9, an achromatic color changes from white through gray to black (at a substantial midpoint in FIG. 9) gradually and continuously, and then changes from black through gray to white gradually and continuously. In an information recording layer 12 shown in FIG. 10, from the left toward the right in FIG. 10, an achromatic color changes from black through gray to while (at a substantial midpoint in FIG. 10) gradually and continuously, and then changes from white through gray to black gradually and continuously, as opposed to the example shown in FIG. 9.

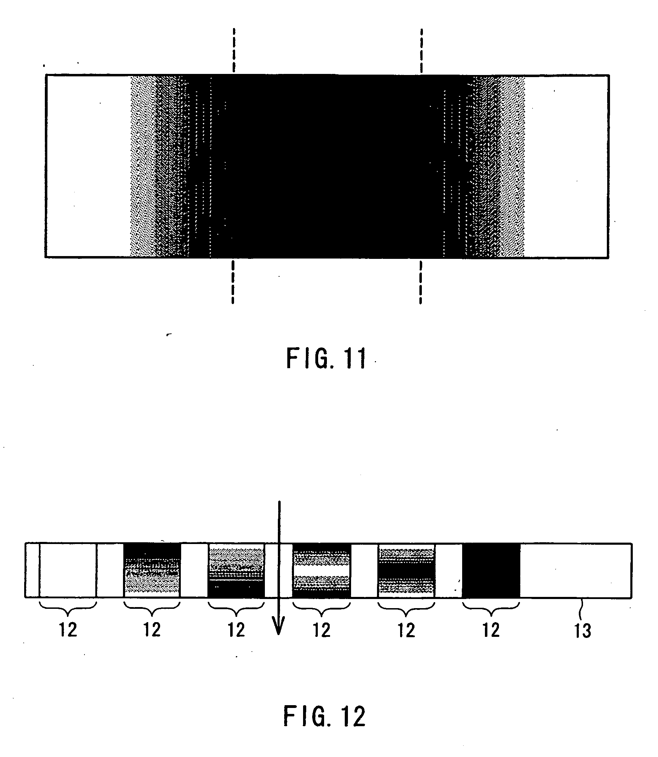

FIG. 11 shows an example of the state where three information recording layers 12 are arranged so as to be in contact with each other with no space at their boundaries, and the intensity of the single color changes continuously between adjacent information recording layers. In these three information recording layers 12, from the left toward the right in FIG. 11, an achromatic color changes from white through gray to black (the information recording layer in the middle) gradually and continuously, and then changes from black through gray to white gradually and continuously.

FIG. 12 shows an example where six information recording layers 12 are arranged on a substrate 13. In this example, as indicated with an arrow, the direction in which information is read out corresponds to the width direction of the substrate 13. As shown in FIG. 12, in this example, only white is used in the information recording layer located first from the left, the intensity of an achromatic color changes continuously along the width direction of the substrate in each of the information recording layers 12 located second to fifth from the left, and only black is used in the information recording layer located sixth from the left.

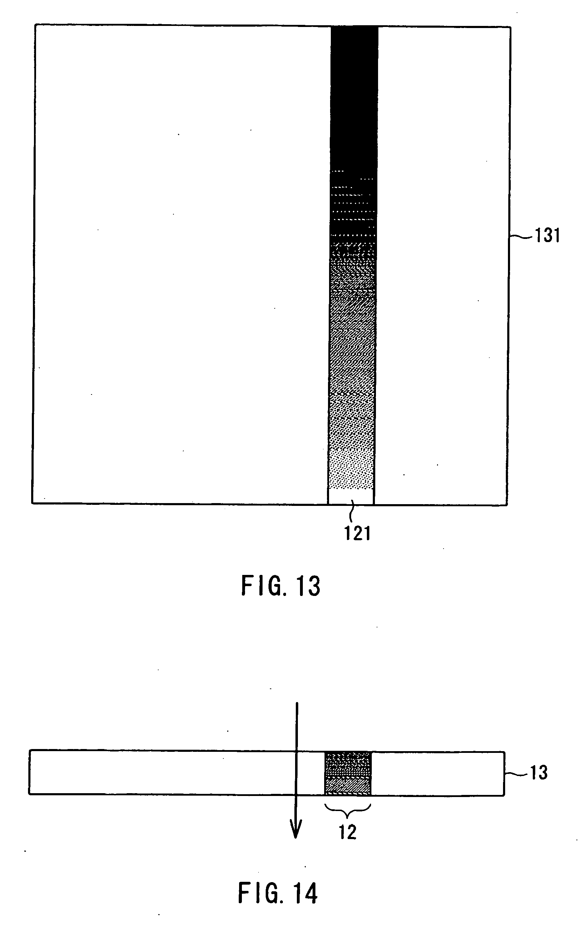

With reference to FIGs. 13 and 14, how to produce a sample analysis tool provided with an information recording layer in which the intensity of a single color changes continuously will be described. In general, a sample analysis tool is produced by cutting out a strip-shaped substrate from a large base film and then arranging a reagent pad(s) (a reagent layer(s)) on the substrate. In the production of the sample analysis tool according to the present invention, as shown in FIG. 13, an information recording part 121 to be an information recording layer is formed on a base film 131 beforehand by printing. Then, by cutting the base film 131 in a strip shape, it is possible to obtain a sample analysis tool composed of a substrate 13 on which an information recording layer 12 is formed as shown in FIG. 14 (no reagent layer is shown in the drawing). In this case, if the information recording part 121 on the base film 131 is formed so that the intensity of a single color changes stepwise distinctively, there is a possibility that, due to a positional deviation caused when cutting the base film 131, information may not be recorded accurately in the information recording layer 12 of the sample analysis tool as a finished product. Thus, taking this into consideration, if the information recording part 121 on the base film 131 is formed so that the intensity changes continuously and gradually at a constant change ratio from black through gray to white from the upper side toward the lower side as shown in FIG. 13, information can be recorded accurately regardless of which portion of the base film 131 is cut out to obtain the sample analysis tool because the change ratio of the intensity is constant in the information recording layer 12. It is to be noted that the change in intensity of the single color in the information recording part 121 or the information recording layer 12 shown in FIGs. 13 and 14 merely is illustrative, and other changes also are applicable in the present invention.

### Industrial Applicability

As specifically described above, in the sample analysis tool of the present invention, the information recording layer serves to allow a sufficient amount of information to be recorded accurately. Besides, since the information can be read out with an ordinary optical system provided in an analyzer, it is not necessary to provide the analyzer with any special mechanism. Therefore, it is expected that the use of the sample analysis tool of the present invention will bring about various advantages such as automation, simplification, and improvement in reliability of tests.

## Claims

1. A sample analysis tool (1) comprising:
a substrate (13);
reagent layers (11); and
an information recording layer (12),
the reagent layers (11) and the information recording layer (12) being formed on the substrate (13),
wherein the information recording layer (12) is composed of one region or divided into a plurality of regions, each region being colored with a single color, and recorded information can be read out optically;
information is recorded using the position of reagent layers and at least one selected from the group consisting of the single color of each region, a combination of color intensities over the plurality of regions, and an area of each region; and
there are a plurality of reagent layers (11) arranged in series;
**characterised in that** pitches between the plurality of reagent layers (11) are constant, except for at least one of a first pitch, a last pitch, and a pitch present between the first pitch and the last pitch, and
this variation in pitch is also used to record the information.

2. The sample analysis tool according to claim 1, wherein the single color is an achromatic color selected from white, gray, and black.

3. The sample analysis tool according to claim 1 or 2, wherein the single color is at least one single color selected from the group consisting of yellow (an absorption wavelength: 350 nm or more and less than 430 nm), blackish brown, red (an absorption wavelength: 430 nm or more and less than 530 nm), purple (an absorption wavelength: 530 nm or more and less than 580 nm), blue (an absorption wavelength: 580 nm or more and less than 640 nm), and green (an absorption wavelength: 640 nm or more and 800 nm or less).

4. The sample analysis tool according to any preceding claim, wherein the information recording layer (12) is divided into two or three regions.

5. The sample analysis tool according to any preceding claim, wherein there are two or more information recording layers (12).

6. The sample analysis tool according to any preceding claim, wherein, with regard to the combination of color intensities over the plurality of regions, a change in intensity of the single color between adjacent ones of the plurality of regions is at least one of a continuous change and a stepwise change.

7. The sample analysis tool according to any preceding claim, wherein there are two or more information recording layers (12) that are in contact with each other, and an intensity of the single color changes continuously between adjacent ones of the information recording layers (12).

8. The sample analysis tool according to claim 5, wherein at least one of the two or more information recording layers (12) is formed between the reagent layers (11).

9. The sample analysis tool according to any preceding claim, wherein the information recorded in the information recording layer is at least one selected from the group consisting of:
information for automatically identifying a provider of the sample analysis tool (1);
information for automatically identifying a country in which the sample analysis tool is sold;
information for automatically identifying the number of test items and an order in which the test items are arranged;
information as to a production batch of the sample analysis tool (a calibration curve or information as to a sensibility of the sample analysis tool);
information as to whether the sample analysis tool (1) is applicable to visual observation only, reading by an analyzer only, or both the visual observation and the reading by an analyzer;
information for identifying a front side (i.e., a reagent side) and a rear side of the sample analysis tool;
information for identifying the presence or absence of a reagent in the sample analysis tool (1);
information for detecting and correcting a positional deviation of the sample analysis tool in an analyzer; and
information for correcting an amount of light when the sample analysis tool is subjected to measurement with an optical system.

10. The sample analysis tool according to claim 1, which is used for a urinalysis, a biochemical test, a microorganism test, an immunological test, a genetic analysis, an environmental test, a test for an agricultural chemical, or an allergen test.

11. The sample analysis tool according to claim 1, wherein the sample analysis tool (1) is used for a urinalysis,
a test item in the urinalysis is at least one selected from the group consisting of glucose (GLU), protein (PRO), bilirubin (BIL), urobilinogen (URO), pH, occult blood (BLD), ketone bodies (KET), nitrite (NIT), leukocytes (LEU), S.G. (specific gravity), a color tone, ascorbic acid, a salt concentration, highly-sensitive protein, albumin, creatinine, Bence-Jones protein, hormones and physiologically active substances, and
at least one reagent layer (11) is provided for each of the test items.

12. The sample analysis tool according to claim 1, which is analyzed by an analyzer.

## Patentansprüche

1. Werkzeug (1) zum Testen einer Probe umfassend:
ein Substrat (13);
Reagenzschichten (11); und
eine Informationsaufzeichnungsschicht (12),
wobei die Reagenzschichten (11) und die Informationsaufzeichnungsschicht (12) auf dem Substrat (13) ausgebildet sind,
wobei die Informationsaufzeichnungsschicht (12) aus einer Region gebildet wird oder in eine Vielzahl von Regionen unterteilt ist, wobei jede Region mit einer Einzelfarbe gefärbt ist, und aufgezeichnete Information optisch ausgelesen werden kann;
wobei Information aufgezeichnet wird, indem die Position von Reagenzschichten und wenigstens einem ausgewählt aus der Gruppe bestehend aus der Einzelfarbe von jeder Region, einer Kombination von Farbintensitäten über die Vielzahl der Regionen, und einem Bereich von jeder Region benutzt wird; und
eine Vielzahl von Reagenzschichten (11) in Serie angeordnet sind;
**dadurch gekennzeichnet, dass** Abstände zwischen der Vielzahl von Reagenzschichten (11) konstant sind, mit Ausnahme von wenigstens einem von einem ersten Abstand, einem letzten Abstand und einem Abstand, der zwischen dem ersten Abstand und dem letzen Abstand besteht, und
diese Abstandsvariation auch benutzt wird, um die Information aufzuzeichnen.

2. Werkzeug zum Testen einer Probe gemäß Anspruch 1, wobei die Einzelfarbe eine achromatische Farbe ausgewählt aus Weiß, Grau und Schwarz ist.

3. Werkzeug zum Testen einer Probe gemäß Anspruch 1 oder 2, wobei die Einzelfarbe wenigstens eine Einzelfarbe ist ausgewählt aus der Gruppe bestehend aus Gelb (eine Absorptionswellenlänge: 350 nm oder mehr und weniger als 430 nm), Schwarzbraun, Rot (eine Absorptionswellenlänge: 430 nm oder mehr und weniger als 530 nm), Violett (eine Absorptionswellenlänge: 530 nm oder mehr und weniger als 580 nm), Blau (eine Absorptionswellenlänge: 580 nm oder mehr und weniger als 640 nm) und Grün (eine Absorptionswellenlänge: 640 nm oder mehr und 800 nm oder weniger).

4. Werkzeug zum Testen einer Probe gemäß einem der vorangegangenen Ansprüche, wobei die Informationsaufzeichnungsschicht (12) in zwei oder drei Regionen unterteilt ist.

5. Werkzeug zum Testen einer Probe gemäß einem der vorangegangenen Ansprüche, wobei es zwei oder mehr Informationsaufzeichnungsschichten (12) gibt.

6. Werkzeug zum Testen einer Probe gemäß einem der vorangegangenen Ansprüche, wobei mit Bezug zu der Kombination von Farbintensitäten über die Vielzahl von Regionen eine Intensitätsänderung der Einzelfarbe zwischen benachbarten der Vielzahl von Regionen wenigstens eines von einem kontinuierlichen Wechsel und einem schrittweisen Wechsel ist.

7. Werkzeug zum Testen einer Probe gemäß einem der vorangegangenen Ansprüche, wobei es zwei oder mehr Informationsaufzeichnungsschichen (12) gibt, die miteinander in Kontakt stehen, und eine Intensität der Einzelfarbe kontinuierlich zwischen Benachbarten der Informationsaufzeichnungsschichten (12) wechselt.

8. Werkzeug zum Testen einer Probe gemäß Anspruch 5, wobei wenigstens eine der zwei oder mehr Informationsaufzeichnungsschichten (12) zwischen den Reagenzschichten (11) ausgebildet ist.

9. Werkzeug zum Testen einer Probe gemäß einem der vorangegangenen Ansprüche, wobei die aufgezeichnete Information in der Informationsaufzeichnungsschicht wenigstens eine ist ausgewählt aus der Gruppe bestehend aus:
Information zum automatischen Identifizieren eines Anbieters des Werkzeugs (1) zum Testen einer Probe;
Information zum automatischen Identifizieren eines Landes, in welchem das Werkzeug zum Testen einer Probe verkauft wird;
Information zum automatischen Identifizieren der Anzahl von Testobjekten und einer Reihenfolge, in welcher die Testobjekte angeordnet sind;
Information bezüglich einer Produktionscharge des Werkzeugs zum Testen einer Probe (eine Kalibrationskurve oder Information bezüglich der Sensibilität des Werkzeugs zum Testen einer Probe);
Information, ob das Werkzeug (1) zum Testen einer Probe anwendbar ist allein zur visuellen Beobachtung, allein zum Lesen durch einen Analysierer, oder sowohl als auch zur visuellen Beobachtung und zum Lesen durch einen Analysierer;
Information zum Identifizieren einer Vorderseite (d.h. einer Reagenzseite) und einer Rückseite des Werkzeugs zum Testen einer Probe;
Information zum Identifizieren der Anwesenheit oder Abwesenheit einer Reagenz in dem Werkzeug (1) zum Testen einer Probe;
Information zum Detektieren und Korrigieren einer Positionsabweichung des Werkzeugs zum Testen einer Probe in einem Analysierer; und
Information zum Korrigieren einer Lichtmenge, wenn das Werkzeug zum Testen einer Probe einer Messung mit einem optischen System unterworfen wird.

10. Werkzeug zum Testen einer Probe gemäß Anspruch 1, welches benutzt wird für eine Harnuntersuchung, einen biochemischen Test, einen Mikroorganismustest, einen immunologischen Test, eine genetische Analyse, einen Umwelttest, einen Test für eine landwirtschaftliche Chemikalie, oder einen Allergentest.

11. Werkzeug zum Testen einer Probe gemäß Anspruch 1, wobei des Werkzeug (1) zum Testen einer Probe für eine Harnuntersuchung benutzt wird,
wobei ein Testobjekt in der Harnuntersuchung wenigstens eines ist ausgewählt aus der Gruppe bestehend aus Glukose (GLU), Protein (PRO), Bilirubin (BIL), Urobilinogen (URO), pH, okkultem Blut (BLD), Ketonkörper (KET), Nitrit (NIT), Leukozyten (LEU), S.G. (spezifischem Gewicht), einem Farbton, Ascorbinsäure, einer Salzkonzentration, hochsensitivem Protein, Albumin, Kreatinin, Bence-Jones Protein, Hormonen und physiologisch aktiven Substanzen, und
wenigstens eine Reagenzschicht (11) für jedes der Testobjekte bereitgestellt wird.

12. Werkzeug zum Testen einer Probe gemäß Anspruch 1, welche durch einen Analysierer analysiert wird.

## Revendications

1. Outil d'analyse d'échantillon (1) comprenant :
un substrat (13) ;
des couches de réactifs (11) ; et
une couche d'enregistrement d'informations (12),
les couches de réactifs (11) et la couche d'enregistrement d'informations (12) étant formées sur le substrat (13),
dans lequel la couche d'enregistrement d'informations (12) est composée d'une seule région ou est divisée en une pluralité de régions, chaque région étant colorée avec une couleur unique, et les informations enregistrées peuvent être lues optiquement ;
les informations sont enregistrées par utilisation de la position des couches de réactifs et d'au moins l'un choisi dans le groupe constitué par la couleur unique de chaque région, une combinaison d'intensités de couleur sur la pluralité de régions, et une zone de chaque région ; et
il y a une pluralité de couches de réactifs (11) disposées en série ;
**caractérisé en ce que** les pas entre la pluralité de couches de réactifs (11) sont constants, sauf pour au moins un premier pas, un dernier pas, et un pas présent entre le premier pas et le dernier pas, et
cette variation de pas est aussi utilisée pour l'enregistrement d'informations.

2. Outil d'analyse d'échantillon selon la revendication 1, dans lequel la couleur unique est une couleur achromatique choisie parmi le blanc, le gris et le noir.

3. Outil d'analyse d'échantillon selon la revendication 1 ou 2, dans lequel la couleur unique est au moins une couleur unique choisie dans le groupe constitué par le jaune (longueur d'onde d'absorption : 350 nm ou plus et inférieure à 430 nm), le marron noirâtre, le rouge (longueur d'onde d'absorption : 430 nm ou plus et inférieure à 530 nm), le pourpre (longueur d'onde d'absorption : 530 nm ou plus et inférieure à 580 nm), le bleu (longueur d'onde d'absorption : 580 nm ou plus et inférieure à 640 nm), et le vert (longueur d'onde d'absorption : 640 nm ou plus et inférieure à 800 nm).

4. Outil d'analyse d'échantillon selon l'une quelconque des revendications précédentes, dans lequel la couche d'enregistrement d'informations (12) est divisée en deux ou trois régions.

5. Outil d'analyse d'échantillon selon l'une quelconque des revendications précédentes, dans lequel il y a deux ou plus de deux couches d'enregistrement d'informations (12).

6. Outil d'analyse d'échantillon selon l'une quelconque des revendications précédentes, dans lequel, en ce qui concerne la combinaison d'intensités de couleur sur la pluralité de régions, un changement d'intensité de la couleur unique entre des régions adjacentes parmi la pluralité de régions est au moins l'un parmi un changement continu et un changement progressif.

7. Outil d'analyse d'échantillon selon l'une quelconque des revendications précédentes, dans lequel il y a deux ou plus de deux couches d'enregistrement d'informations (12) qui sont en contact les unes avec les autres, et une intensité de la couleur unique change en continu entre des couches adjacentes parmi les couches d'enregistrement d'informations (12).

8. Outil d'analyse d'échantillon selon la revendication 5, dans lequel au moins l'une des deux ou plus de deux couches d'enregistrement d'informations (12) est formée entre les couches de réactifs (11).

9. Outil d'analyse d'échantillon selon l'une quelconque des revendications précédentes, dans lequel les informations enregistrées dans la couche d'enregistrement d'informations sont au moins un type d'informations choisi dans le groupe constitué par :
des informations pour identifier automatiquement le fournisseur de l'outil d'analyse d'échantillon (1) ;
des informations pour identifier automatiquement le pays dans lequel l'outil d'analyse d'échantillon est vendu ;
des informations pour identifier automatiquement le nombre d'objets de test et l'ordre dans lequel les objets de test sont disposés ;
des informations concernant le lot de production de l'outil d'analyse d'échantillon (une courbe d'étalonnage ou des informations concernant la sensibilité de l'outil d'analyse d'échantillon) ;
des informations sur l'applicabilité de l'outil d'analyse d'échantillon (1) à une observation visuelle uniquement, à une lecture par un analyseur uniquement, ou à la fois à une observation visuelle et à une lecture par un analyseur ;
des informations pour identifier le côté recto (c'est-à-dire le côté réactifs) et le côté verso de l'outil d'analyse d'échantillon ;
des informations pour identifier la présence ou l'absence d'un réactif dans l'outil d'analyse d'échantillons (1) ;
des informations pour détecter et corriger une déviation de position de l'outil d'analyse d'échantillon dans un analyseur ; et
des informations pour corriger la quantité de lumière quand l'outil d'analyse d'échantillon est soumis à une mesure avec un système optique.

10. Outil d'analyse d'échantillon selon la revendication 1, qui est utilisé pour une analyse d'urine, un test biochimique, un test de micro-organisme, un test immunologique, une analyse génétique, un test environnemental, un test pour un produit chimique à usage agricole, ou un test d'allergène.

11. Outil d'analyse d'échantillon selon la revendication 1, lequel outil d'analyse d'échantillon (1) est utilisé pour une analyse d'urine,
un objet de test dans l'analyse d'urine est au moins un objet choisi dans le groupe constitué par le glucose (GLU), une protéine (PRO), la bilirubine (BIL), l'urobilinogène (URO), le pH, une hémorragie occulte (BLD), des corps cétoniques (KET), un nitrite (NIT), les leucocytes (LEU), le poids spécifique (S.G.), une nuance de couleur, l'acide ascorbique, la concentration d'un sel, une protéine très sensible, l'albumine, la créatinine, la protéine de Bence-Jones, les hormones et les substances physiologiquement actives, et
au moins une couche de réactifs (11) est prévue pour chacun des objets de test.

12. Outil d'analyse d'échantillon selon la revendication 1, qui est analysé par un analyseur.
